# EUROPEAN PATENT SPECIFICATION

(11) **EP 3 318 244 B1**
(45) Date of publication and mention of the grant of the patent: **15.04.2020**
(21) Application number: 17207411.4
(22) Date of filing: 30.03.2015
(51) Int. Cl.: A61K 8/26, A61K 8/37, A61Q 3/02, A61K 8/81, A61K 8/04, A45D 29/00, A45D 34/04

(54) **A COMPOSITION OF A DECORATIVE POLISH FOR FINGERNAILS AND A METHOD OF DECORATING FINGERNAILS WITH SAID POLISH**
ZUSAMMENSETZUNG EINES DEKORATIVEN POLIERMITTELS FÜR FINGERNÄGEL UND VERFAHREN ZUM VERZIEREN VON FINGERNÄGELN MIT BESAGTEM POLIERMITTEL
COMPOSITION DE PRODUIT DE POLISSAGE DÉCORATIF POUR ONGLES ET PROCÉDÉ DE DÉCORATION D'ONGLES AVEC LEDIT PRODUIT DE POLISSAGE

(30) Priority: 02.04.2014 IT MI20140580
(43) Date of publication of application: 09.05.2018
(62) Divisional of application: 15161763.6
(73) Proprietor: Chromavis S.p.A., 20122 Milano (IT)
(72) Inventor: WAJSGROS, Emilie, 75012 PARIS (FR); MARTINET, Mandy, 92120 Montrouge (FR)
(74) Representative: Vanosi, Adelio Valeriano

(56) References cited:
- JP-A- S55 139 316
- JP-A- 2002 234 821
- US-A- 5 427 121
- US-A1- 2001 037 750

## Description

### FIELD OF THE INVENTION

The present invention relates to a composition of a decorative polish for fingernails and to a method of decorating fingernails with said polish.

### STATE OF THE ART

The prior art for decorating fingernails encompasses the application of a layer of coloured, transparent, pearly, etc. nail polish on the fingernails by using a suitable brush.

The application of the coloured fingernail polish requires good manual dexterity and accuracy in order to avoid the undesired colouring also of the cuticles or portions of skin surrounding the nail, which causes an unaesthetic effect. When the fingernail polish accidentally runs on the skin or cuticles surrounding the nail, the removal exclusively of the accidentally coloured skin or cuticles is quite laborious, so that most of the times it is more practical to use a solvent (e.g. acetone, or mixtures of esters such as ethylacetate and butylacetate...) and completely remove the fingernail polish from both skin/cuticles and fingernail. In these cases, it is then necessary to repeat the application.

Often, in order to obtain special effects, after having applied the coloured polish base, artistic, geometric or pattern designs are drawn on said base by using a thin brush to revive the base with pearlescent, iridescent or glittered visual effects through the application of a further polish layer generating such effects.

Fingernail polishes are also known, for which the application of a transparent polish base layer is encompassed, which reinforces and regenerates the fingernail, and then the application of a coloured layer, which gives the desired colour to the fingernail, on which a top coat may be added, thus acting as a fixative, shining aid and long-lasting enhancer.

In this case, the polish base is transparent or slightly coloured in pastel or nude shades and its application is not particularly difficult since any smear is not visible. On the other hand, the application of the coloured polish layer has the same difficulties as found in the application of a coloured polish used alone.

An error in the application of the colouring layer can be corrected as above with the aid of acetone or suitable solvents, which however in this case also remove the base layer. It is thus necessary to re-apply both the base and the coloured polish.

JP 2002 234821 A discloses a solvent-type nail cosmetic that is free of inorganic pigment sedimentation and has excellent aging stability, film properties and usability.

JP S55 139316 A discloses a nail polish that can form multicoloured patterns. It comprises a combination of a base enamel and a facing liquid.

US patent 5,427,121 is also known, which describes a method of applying a nail polish to the fingernails comprising the steps of applying a polish base on the fingernail by using a conventional brush, spraying at least one layer of acrylic paint on the fingernail, applying a "top coat" on the acrylic paint sprayed thereon by using a brush so as to fix the colour of the acrylic paint on the nail, and removing the residues of acrylic paint from the cuticles and from the parts of the skin surrounding the fingernail by washing with water and soap.

The method described in the above patent has several drawbacks.

In fact, after having sprayed the acrylic paint on the fingernail pre-treated with a polish base, it is necessary to apply a transparent fixative with a conventional brush and wait a few minutes for drying. This considerably extends the waiting time to see the finished result: it is necessary to wait for the drying of the base, the drying of the colouring solution and finally the drying of the colour fixative.

The application does not introduce any advantage over the traditional nail polish and does not completely solve the problems of possible smears. In fact, when the brush soaked with fixative is brushed on the paint previously sprayed, the latter dissolves the sprayed paint, thus blending therewith, and thus generating possible smears if applied awkwardly.

Moreover, the colour of the paint blended with the fixative which remains on the brush contaminates the fixative contained in the bottle which the brush is dipped in, thus making it unusable for next applications with different colours.

It is therefore the object of the present invention to provide a decorative polish for fingernails and a method of decorating them by means of such a polish, which solves the technical problems described in the prior art.

It is a further object of the present invention to reduce the time required for the application of the decorative nail polish, thus making the application faster, more flexible, practical and effective, compared to both the traditional procedure and the procedure described in US5427121.

### SUMMARY OF THE INVENTION

The above objects have been achieved by a decorative polish for fingernails and by a method of decorating them by means of such a polish, as implemented according to the technical teachings of the appended claims.

The invention relates to a decorative nail polish which is applied by means of a spray cylinder with no particular care to accurately distribute the polish only on the fingernails.

The characteristics and the advantages of the present invention will become apparent from the following detailed description and from the working examples provided for illustrative purposes and from the accompanying Figures, wherein:
- figure 1 is a diagrammatic view of a first step of the nail decoration method according to the present invention;
- figures 2A and 2B diagrammatically show a second step of a nail decoration method obtained in various ways, in which Figure 2A shows a decorative polish according to the present invention being applied; and
- figure 3 shows the final result obtained that, after the step of figure 2A, is by means of the method of the present invention.

### DETAILED DESCRIPTION OF THE INVENTION

The subject of the invention is therefore a composition of a decorative liquid nail polish, and a method of decorating nails by means of such a polish.

The above-mentioned figures show the hand of a user and in particular the nails to be painted or decorated, globally indicated with reference number 1.

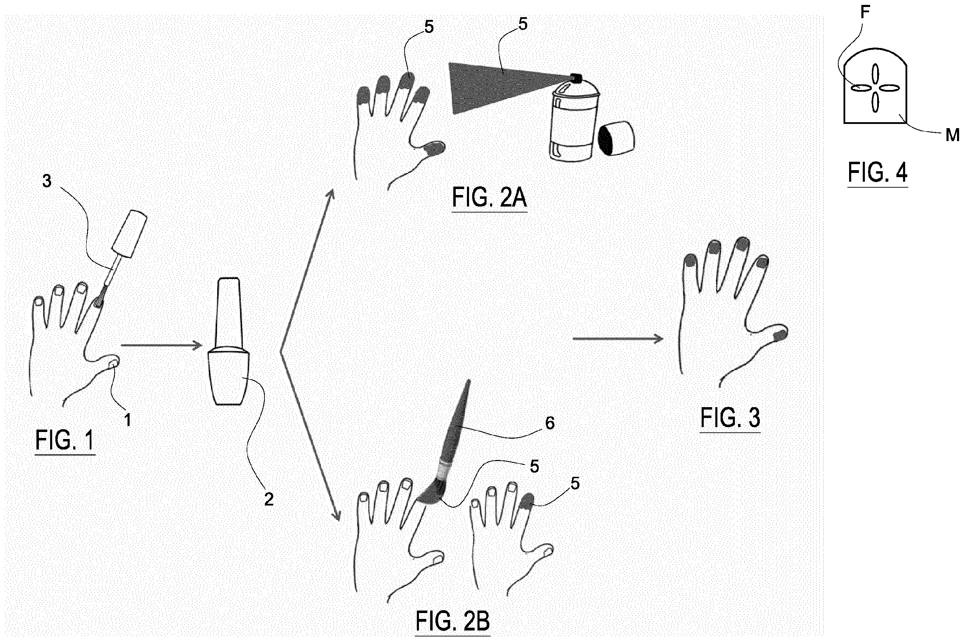

In a first step, as shown in Figure 1, a first layer of transparent (or coloured, as it will be described later) base layer 2 is applied on nails 1 in a traditional manner, i.e. by using a brush 3. The operation should be carried out with the utmost care, in order to avoid covering the cuticles or the skin with the polish base.

Once the first layer has completely dried, a second layer of decorative polish 3 made according to the invention described hereafter is applied on the first layer of base polish 2. The second layer of decorative polish can be applied by using a standard nail brush or a large brush 6 (even larger than the nail, as shown in Figure 2B) or, in accordance with the invention, by means of a spray applicator (Figure 2A).

In other words, the decorative polish can be applied without high accuracy but quickly, without taking care to apply it only on the nail but also let it run on the cuticles and on the skin surrounding the nail itself, thus obtaining the final result shown in Figure 3.

This is particularly advantageous when the final colour or the final finishing of the nail is not yet decided or when there is little time to dedicate to the nail painting. Actually, an accurate operation of the base application can be carried out when the necessary time is available for performing a flawless operation, for example, with a neutral polish.

At a later time, the nail can be given a custom finish or colour in a very short time (a few seconds, just the time to spray the decorative spray polish and let it dry) and with innovative gestures.

The invention is particularly advantageous for painting the toenails which, as known, requires more uncomfortable gestures than the application of the polish on the fingernails.

With reference to Figure 4, alternatively, a decorated template M may be used, for example with windows F arranged in the shape of flower petals, patterns of any type, letters or the like. The template is placed on the nail and the decorative polish is sprayed or applied thereon as described above.

According to the present invention, the decorative polish contains a large amount of solvent, i.e. from 75% to 90%, and a very small (less than 3%) amount of fixative, such as resins. The solvent allows to dissolve the surface of the polish base, and in particular the resins therein, and to fix the decorative component present in the decorative polish (after solvent evaporation, which occurs in about 20 seconds) only in that part already covered with the polish base.

In fact, the percentage of fixative (preferably a resin, e.g. an acrylate copolymer) present in the decorative polish is not sufficient to allow the decorative component to be fixed also on parts of the skin and cuticles without polish base.

Therefore, it is enough to wash with soap and water after the complete evaporation of the solvent of the decorative polish to perfectly remove any trace thereof from all those parts of the skin or cuticles where the polish base is not present. In fact, the decorative polish preferably has a formulation which makes it water-soluble, even after the evaporation of the solvent contained therein.

Preferably, the solvent used in the polish base is ethyl acetate, which when evaporates does not mat the surface of the polish base, thus leaving it bright and homogeneous.

It should be noted that the invention is extremely versatile and is suitable for various uses. In fact, the polish base may be of the transparent type, while the decorative polish may be coloured.

Alternatively, the polish base may be coloured while the decorative polish may add pearlescent, iridescent or glittered visual effects.

In general terms, the composition of the decorative polish, which is in liquid form, comprises:
- at least one solvent, in a percentage of 75 to 90%,
- at least one fixer/film-forming agent, in a percentage of 0.5 to 3%,
- at least one colorant, in a percentage of 5 to 20%,
   and optionally
- at least one perfume, in a percentage of 0.1 to 5%,
- at least one additive, in a percentage of 0.1 to 5%, said percentages being expressed by weight.

Unless otherwise defined, the percentages for the purposes of the present invention are wt% on the weight of the polish composition.

With the expression "fixer/film-forming agent" or "fixer" it is meant a fixative, which acts also as a film-forming agent onto the nail.

Said at least one solvent is preferably an organic solvent.

More preferably, said organic solvent is selected from those belonging to the following chemical classes and are preferably those indicated in parentheses:
- esters (ethylacetate, buthylacetate, etc.),
- alcohols (ethanol, n-butanol, isopropyl alcohol, etc.),
- hydrocarbons (isododecane, etc.),
- ketones (acetone),
- silicone-based solvents (silanes and siloxanes such as cyclomethicone, etc.)

As already mentioned above, the preferred solvent is ethyl acetate, which when evaporates does not mat the surface of the polish base, thus leaving it bright and homogeneous.

Said at least one fixative is preferably selected from the following chemical classes, the more preferred being in parentheses:
- synthetic polymers (acrylate copolymer, polybutene, VP/VA copolymer, polyurethane, hydrogenated polycyclopentadiene, etc.)
- silicone resins (trimethylsiloxysilicate, polymethylsilsesquioxane, C26-28 alkyl dimethicone)
- ether-based resins (polyvinyl stearyl ether, etc.),

Water-soluble fixatives are even more preferable. Water-soluble acrylate copolymers are highly preferred.

In preferred embodiments, said at least one solvent is selected from the group consisting of ethyl acetate, butyl acetate, ethanol, n-butanol, isopropyl alcohol, isododecane, acetone, cyclomethicone, and mixtures thereof, said at least one fixative is water-soluble and selected from the group consisting of acrylate copolymer, polybutene, VP/VA copolymer, polyurethane, hydrogenated polycyclopentadiene, trimethylsiloxysilicate, polymethylsilsesquioxane, C26-28 alkyl dimethicone, polyvinyl stearyl ether, and mixtures thereof.

Said at least one additive can be tocopherol, D-panthenol, ascorbyl palmitate or a mixture thereof.

Said at least one colorant is preferably selected from the following types:
- pigments (iron oxide, titanium oxide, carmine red, barium-based lacquer, calcium-based lacquer, aluminum-based lacquer, etc.), all preferably treated with triethoxycaprylylsilane if an opaque colour effect is desired,
- pearls or pearlescent elements (on a support of natural mica, synthetic mica, alumina, aluminum, CaAl borosilicate, synthetic fluorphlogopite, borosilicate, polyethylene terephthalate, etc.) for a bright and iridescent result,
- natural or synthetic white powders (talc, kaolin, silica, nylon-6, PMMA, etc.) for a velvety effect.

In preferred embodiments, said at least one colorant is selected from the group consisting of pigments, pearls, pearlescent elements, white natural powders, white synthetic powders, and mixtures thereof.

In more preferred embodiments, said at least one colorant is selected from the group consisting of iron oxide, titanium oxide, carmine red, aluminum-based lacquer, calcium-based lacquer, barium-based lacquer, calcium-based lacquer, all preferably treated with triethoxycaprylylsilane, pearls or pearlescent elements on a support of natural mica, synthetic mica, alumina, aluminum, CaAl borosilicate, fluorphlogopite, borosilicate, polyethylene terephthalate, talcum, kaolin, silicon, nylon-6, PMMA, and mixtures thereof. The choice of the colorants will be advantageously made according to the result and to the type of decorative polish to be obtained.

If a colouring decorative polish is desired, to be distributed on a neutral polish base, for example, pigments and also more elements belonging to the pigment category will mainly be used.

On the other hand, if a pearly decorative polish is desired, to be used preferably on a coloured base, pearls or pearlescent elements or a mixture of elements belonging to this category will mainly be used.

On the other hand, if a decorative polish with a velvety touch and a matt/dusty effect is desired, to be used preferably on a transparent polish base, white powders or a mixture of elements belonging to this category will mainly be used.

It will also be possible to mix together more elements of different types.

The decorative polish made as indicated above is in a liquid form at room temperature (about 25°C) and is directly applicable on the polish base.

The decorative polish is distributed on top of the polish base by using a spray applicator. The nail decoration operation is very fast and simple. Advantageously, the coloured surface is perfectly smooth and free of any streaks resulting from the use of a brush.

The propellant may belong to one of the following chemical classes:
- hydrocarbons (butane, propane, etc.),
- ethers (dimethyl ether),
- halogen compounds (difluoroethane).

The preferred substances are indicated in parentheses.

Preferably, the propellant is selected from the group consisting of butane, propane, dimethyl ether, difluoroethane, and mixtures thereof.

In an embodiment, the spray applicator is of the type with deformable bag.

In this case, the decorative liquid polish is contained in a deformable bag in turn inserted into the chamber of a spray cylinder. The bag is in communication with a dispensing valve as in the case described above.

In this embodiment, however, the propellant is not dissolved in the decorative liquid polish but is contained under pressure outside the bag, in the chamber of the spray cylinder.

In this embodiment, the gas preferably used is pressurized nitrogen.

In a different embodiment, the decorative liquid polish is contained in a deformable bag tightly connected to a hermetic pump associated with an atomizer-type actuation and dispensing button.

An embodiment of the decorative liquid polish includes:
- 89% ethylacetate,
- 1% water-soluble copolymer acrylate, and
- 10% natural mica.

The resulting decorative liquid polish is pearlescent.

In another aspect, the present invention concerns the use of a of a decorative liquid polish comprising:
- at least one solvent, in a percentage of 75 to 90%,
- at least one fixative, in a percentage of 0.5 to 3%,
- at least one colorant, in a percentage of 5 to 20%,
   and optionally
- at least one perfume, in a percentage of 0.1 to 5%,
- at least one additive, in a percentage of 0.1 to 5%,
   said percentages being expressed by weight,
   for the production of a sprayable decorative nail polish according to claim 1.

The present invention also relates to a method of decorating nails comprising the step of applying a layer of the sprayable decorative polish as above described, at least on top of a polish base previously applied on the fingernail.

Preferably, the sprayable decorative polish is applied by spraying it on the polish base previously applied on the fingernail.

More preferably, the method of decorating nails comprises:
a) distributing a polish base on a nail and letting it dry,
b) applying a sprayable decorative polish on at least a part of the finger and nail, which comprises:
   - at least one solvent in a percentage of solvents of 75 to 90 wt%, to dissolve the fixatives of the polish base,
   - a colorant, and
   - fixatives in a percentage of 0.5 to 3 wt%,
c) letting the decorative polish dry,
d) washing the nail and finger with water to remove the excess decorative polish on the finger.

It should be understood that all the combinations of preferred aspects of the sprayable decorative nail polish, as well as the method of applying the same, as above reported, are to be deemed as hereby disclosed.

## Claims

1. A sprayable decorative nail polish comprising a decorative liquid polish, said decorative liquid polish comprising:
- at least one solvent, in a percentage of 75 to 90%,
- at least one fixative, in a percentage of 0.5 to 3%,
- at least one colorant, in a percentage of 5 to 20%,
and optionally
- at least one perfume, in a percentage of 0.1 to 5%,
- at least one additive, in a percentage of 0.1 to 5%,
said percentages being expressed by weight,
wherein said decorative liquid polish is contained in a deformable bag of a spray applicator, the bag being in communication with a dispensing valve and the bag being contained in a chamber of a spray cylinder with a propellant outside the bag, or wherein the bag is tightly connected to a hermetic pump associated with an atomizer-type actuation and dispensing button.

2. The sprayable decorative nail polish of claim 1, wherein said at least one solvent is selected from the group consisting of ethyl acetate, butyl acetate, ethanol, n-butanol, isopropyl alcohol, isododecane, acetone, cyclomethicone, and mixtures thereof, said at least one fixative is water-soluble and selected from the group consisting of acrylate copolymer, polybutene, VP/VA copolymer, polyurethane, hydrogenated polycyclopentadiene, trimethylsiloxysilicate, polymethylsilsesquioxane, C26-28 alkyl dimethicone, polyvinyl stearyl ether, and mixtures thereof.

3. The sprayable decorative nail polish of claim 1 or 2, wherein said at least one colorant is selected from the group consisting of pigments, pearls, pearlescent elements, white natural powders, white synthetic powders, and mixtures thereof, and/or wherein said at least one colorant is selected from the group consisting of iron oxide, titanium oxide, carmine red, aluminum-based lacquer, calcium-based lacquer, barium-based lacquer, calcium-based lacquer, all preferably treated with triethoxycaprylylsilane, pearls or pearlescent elements on a support of natural mica, synthetic mica, alumina, aluminum, CaAl borosilicate, fluorphlogopite, borosilicate, polyethylene terephthalate, talcum, kaolin, silicon, nylon-6, PMMA, and mixtures thereof.

4. The sprayable decorative nail polish of any one of claim 1- 3, wherein at least one additive can be tocopherol, D-panthenol, ascorbyl palmitate or a mixture thereof.

5. The sprayable decorative nail polish of any one of claim 1 - 5, comprising:
- 25 to 35% of the decorative liquid polish, and
- 65 to 75% of a propellant,
the percentages being expressed by weight.

6. The sprayable decorative nail polish of any one of claim 1- 5, wherein the propellant is selected from the group consisting of butane, propane, dimethyl ether, difluoroethane, and mixtures thereof.

7. Use of a decorative liquid polish comprising:
- at least one solvent, in a percentage of 75 to 90%,
- at least one fixative, in a percentage of 0.5 to 3%,
- at least one colorant, in a percentage of 5 to 20%,
and optionally
- at least one perfume, in a percentage of 0.1 to 5%,
- at least one additive, in a percentage of 0.1 to 5%,
said percentages being expressed by weight,
for the production of a sprayable decorative nail polish according to claim 1.

8. A method of decorating at least one fingernail, comprising the step of applying a layer of the sprayable decorative nail polish of any one of claim 1- 6, at least on top of a polish base previously applied on the fingernail.

9. The method of claim 8, wherein the sprayable decorative nail polish is applied by spraying it on the polish base previously applied on the fingernail.

## Patentansprüche

1. Sprühbarer dekorativer Nagellack, umfassend einen dekorativen Flüssiglack, der dekorative Flüssiglack umfassend:
- mindestens ein Lösungsmittel in einem Prozentsatz von 75 bis 90 %,
- mindestens ein Fixiermittel in einem Prozentsatz von 0,5 bis 3 %,
- mindestens einen Farbstoff in einem Prozentsatz von 5 bis 20 % und optional
- mindestens ein Parfüm in einem Prozentsatz von 0,1 bis 5 %,
- mindestens einen Zusatzstoff in einem Prozentsatz von 0,1 bis 5 %, wobei die Prozentsätze nach Gewicht ausgedrückt sind, wobei der dekorative Flüssiglack in einem verformbaren Beutel eines Sprühapplikators enthalten ist, wobei der Beutel in Verbindung mit einem Ausgabeventil ist und der Beutel in einer Kammer eines Sprühzylinders mit einem Treibmittel außerhalb des Beutels enthalten ist, oder wobei der Beutel fest mit einer hermetischen Pumpe verbunden ist, die mit einem Betätigungs- und Abgabeknopf vom Zerstäubertyp verbunden ist.

2. Sprühbarer dekorativer Nagellack nach Anspruch 1, wobei das mindestens eine Lösungsmittel ausgewählt ist aus der Gruppe bestehend aus Ethylacetat, Butylacetat, Ethanol, n-Butanol, Isopropylalkohol, Isododecan, Aceton, Cyclomethicon und Gemischen davon, wobei das mindestens eine Fixiermittel wasserlöslich ist und ausgewählt ist aus der Gruppe bestehend aus Acrylat-Copolymer, Polybuten, VP/VA-Copolymer, Polyurethan, hydriertem Polycyclopentadien, Trimethylsiloxysilikat, Polymethylsilsesquioxan, C26-28-Alkyldimethicon, Polyvinylstearylether und Gemischen davon besteht.

3. Sprühbarer dekorativer Nagellack nach Anspruch 1 oder 2, wobei der mindestens eine Farbstoff ausgewählt ist aus der Gruppe bestehend aus Pigmenten, Perlen, Perlglanzelementen, weißen natürlichen Pulvern, weißen synthetischen Pulvern und Gemischen davon, und/oder wobei der mindestens eine Farbstoff ausgewählt ist aus der Gruppe bestehend aus Eisenoxid, Titanoxid, Karminrot, Lack auf Aluminiumbasis, Lack auf Calciumbasis, Lack auf Bariumbasis, Lack auf Calciumbasis, alles vorzugsweise behandelt mit Triethoxycaprylylsilan, Perlen oder Perlglanzelementen auf einem Träger aus natürlichem Glimmer, synthetischem Glimmer, Aluminiumoxid, Aluminium, CaAl-Borosilikat, Fluorphlogopit, Borosilikat, Polyethylenterephthalat, Talkum, Kaolin, Silizium, Nylon-6, PMMA und Gemischen davon.

4. Sprühbarer dekorativer Nagellack nach einem der Ansprüche 1-3, wobei mindestens ein Zusatzstoff Tocopherol, D-Panthenol, Ascorbylpalmitat oder ein Gemisch davon sein kann.

5. Sprühbarer dekorativer Nagellack nach einem der Ansprüche 1-5, umfassend:
- 25 bis 35 % des dekorativen Nagellacks, und
- 65 bis 75 % eines Treibmittels, wobei die Prozentsätze nach Gewicht ausgedrückt sind.

6. Sprühbarer dekorativer Nagellack nach einem der Ansprüche 1-5, wobei das Treibmittel ausgewählt ist aus der Gruppe bestehend aus Butan, Propan, Dimethylether, Difluorethan und Gemischen davon.

7. Verwendung eines dekorativen Flüssiglack, umfassend:
- mindestens ein Lösungsmittel in einem Prozentsatz von 75 bis 90 %,
- mindestens ein Fixiermittel in einem Prozentsatz von 0,5 bis 3 %,
- mindestens einen Farbstoff in einem Prozentsatz von 5 bis 20 % und optional
- mindestens ein Parfüm in einem Prozentsatz von 0,1 bis 5 %,
- mindestens einen Zusatzstoff in einem Prozentsatz von 0,1 bis 5 %, wobei die Prozentsätze nach Gewicht ausgedrückt sind, für die Herstellung eines sprühbaren dekorativen Nagellacks nach Anspruch 1.

8. Verfahren zum Dekorieren mindestens eines Fingernagels, umfassend den Schritt eines Auftragens einer Schicht des sprühbaren dekorativen Nagellacks nach einem der Ansprüche 1 bis 6, mindestens auf eine vorher auf den Fingernagel aufgetragene Lackbasis.

9. Verfahren nach Anspruch 8, wobei der sprühbare dekorative Nagellack durch Sprühen auf die vorher auf den Fingernagel aufgetragene Lackbasis aufgetragen wird.

## Revendications

1. Vernis à ongles décoratif pulvérisable comprenant un vernis liquide décoratif, ledit vernis liquide décoratif comprenant :
- au moins un solvant, à raison de 75 à 90 %,
- au moins un fixatif, à raison de 0,5 à 3 %,
- au moins un colorant, à raison de 5 à 20 %,
et optionnellement
- au moins un parfum, à raison de 0,1 à 5 %,
- au moins un additif, à raison de 0,1 à 5 %,
les pourcentages étant exprimés en poids,
ledit vernis liquide décoratif étant contenu dans un sac déformable d'un applicateur pulvérisateur, le sac étant en communication avec une valve de distribution et le sac étant contenu dans une chambre d'un cylindre de pulvérisation avec un propulseur à l'extérieur du sac, ou le sac étant étroitement relié à une pompe hermétique associée avec un bouton d'actionnement et de distribution de type atomiseur.

2. Vernis à ongles décoratif pulvérisable selon la revendication 1, dans lequel ledit ou lesdits solvants sont sélectionnés dans le groupe constitué de : acétate d'éthyle, acétate de butyle, éthanol, n-butanol, alcool isopropylique, isododécane, acétone, cyclométhicone, et des mélanges de ceux-ci, ledit ou lesdits fixatifs sont solubles dans l'eau et sélectionnés dans le groupe constitué de : copolymère acrylate, polybutène, copolymère VP/VA, polyuréthane, polycyclopentadiène hydrogéné, triméthylsiloxysilicate, polyméthylsilsesquioxane, diméthicone d'alkyle C26-28, éther de stéaryle polyvinylique, et des mélanges de ceux-ci.

3. Vernis à ongles décoratif pulvérisable selon la revendication 1 ou 2, dans lequel ledit ou lesdits colorants sont sélectionnés dans le groupe constitué de : pigments, perles, éléments nacrés, poudres naturelles blanches, poudres synthétiques blanches, et mélanges de ces éléments, et/ou dans lequel ledit ou lesdits colorants sont sélectionnés dans le groupe constitué de : oxyde de fer, oxyde de titane, rouge carmin, laque à base d'aluminium, laque à base de calcium, laque à base de baryum, laque à base de calcium, tous traités de préférence avec du triéthoxycaprylylsilane, des perles ou des éléments nacrés sur un support de mica naturel, mica synthétique, alumine, aluminium, borosilicate de CaAl, fluorphlogopite, borosilicate, téréphtalate de polyéthylène, talc, kaolin, silicium, nylon-6, PMMA, et des mélanges de ces éléments.

4. Vernis à ongles décoratif pulvérisable selon l'une quelconque des revendications 1-3, dans lequel au moins un additif peut être : tocophérol, D-panthénol, palmitate d'ascorbyle ou un mélange de ceux-ci.

5. Vernis à ongles décoratif pulvérisable selon l'une quelconque des revendications 1-5, comprenant :
- 25 à 35 % du vernis liquide décoratif, et
- 65 à 75 % d'un propulseur,
les pourcentages étant exprimés en poids.

6. Vernis à ongles décoratif pulvérisable selon l'une quelconque des revendications 1-5, dans lequel le propulseur est sélectionné dans le groupe constitué de butane, propane, éther diméthylique, difluoroéthane, et des mélanges de ceux-ci.

7. Utilisation d'un vernis liquide décoratif comprenant :
- au moins un solvant, à raison de 75 à 90 %,
- au moins un fixatif, à raison de 0,5 à 3 %,
- au moins un colorant, à raison de 5 à 20 %,
et optionnellement
- au moins un parfum, à raison de 0,1 à 5 %,
- au moins un additif, à raison de 0,1 à 5 %,
les pourcentages étant exprimés en poids,
pour la production d'un vernis à ongles décoratif pulvérisable selon la revendication 1.

8. Procédé de décoration d'au moins un ongle, comprenant l'étape d'application d'une couche du vernis à ongles décoratif pulvérisable selon l'une quelconque des revendications 1-6, au moins par-dessus une base de vernis préalablement appliquée sur l'ongle.

9. Procédé selon la revendication 8, dans lequel le vernis à ongles décoratif pulvérisable est appliqué par pulvérisation sur la base de vernis préalablement appliquée sur l'ongle.
